# EUROPÄISCHE PATENTANMELDUNG

(11) **EP 1 138 387 A1**
(43) Veröffentlichungstag der Anmeldung: **04.10.2001**
(21) Anmeldenummer: 00106671.1
(22) Anmeldetag: 29.03.2000
(51) Int. Cl.: B01J 29/04, C07D 301/12, C07C 249/04

(54) **Verfahren zur Herstellung eines Titansilicalitformkörpers**

(71) Anmelder: Degussa AG, 40474 Düsseldorf (DE)
(72) Erfinder: Hasenzahl, Steffen, Dr., 63477 Maintal (DE); Jantke, Ralf, 63776 Mömbris (DE)
(74) Vertreter: Sternagel, Fleischer, Godemeyer & Partner Patentanwälte

(57) **Zusammenfassung**

Die vorliegende Erfindung betrifft ein Verfahren zur Herstellung eines Titansilicalitformkörpers durch
a) Ausbilden einer verformbaren Masse enthaltend Titansilicalit, ein Bindemittel und ein Anteigungsmittel, so daß die Curdkurve der verformbaren Masse einen Plateauwert im Bereich von 20 bis 90 mm aufweist,
b) Verformen der Masse aus Schritt a) zu einem Grünkörper,
c) eventuell Trocknen und
d) Calcinieren des Grünkörpers, einen nach diesem Verfahren erhältlichen Titansilicalitformkörper und die Verwendung dieser Titalsilicalitformkörper zur Epoxidierung von Olefine oder Ammoximierung von Ketonen.

## Beschreibung

Die vorliegende Erfindung betrifft ein Verfahren zur Herstellung eines Titansilicalitformkörpers, einen Titansilicalitformkörper erhältlich nach dem oben beschriebenen Verfahren und Verfahren zur Epoxidierung von Olefinen sowie zur Ammoximierung von Ketonen unter Verwendung dieses Titansilicalitformkörpers.

Aus US-A-4,410,501 ist ein Verfahren zur Herstellung von Titansilicalit wie auch die Verwendung des so hergestellten Titansilicalits als Katalysator in einer Reihe von Reaktionen, u. a. Oxidationsreaktionen, bekannt. Die Herstellung des Titansilicalits erfolgt durch Ausbildung eines Synthesegels, ausgehend von einer hydrolysierbaren Siliciumverbindung wie z. B. Tetraethylorthosilicat und einer hydrolysierbaren Titanverbindung durch Zugabe von Tetra-n-propylammoniumhydroxid, Hydrolysieren und Kristallisieren dieser Reaktionsmischung. Nach Beendigung der Kristallisation werden die Kristalle durch Filtration abgetrennt, gewaschen, getrocknet und schließlich 6 h lang bei 550°C calciniert.

In DE-A 197 31 672 ist ein Verfahren zur Herstellung von Titansilicalitgranulaten beschrieben, wonach ein Synthesegel, das eine SiO₂-Quelle, eine TiO₂-Quelle Tetra-n-propylammoniumionen enthaltende Verbindung, eine Base und Wasser enthält unter hydrothermalen Bedingungen kristallisiert wird und danach die dabei entstehende Titansilicalitsuspension ohne vorherige Abtrennung einer Sprühtrocknung oder einer Wirbelschichtsprühgranulationstrocknung unterzogen wird, und danach das gebildete Titansilicalitgranulat bei einer Temperatur von 400°C bis 1.000°C, vorzugsweise von 500°C bis 750°C calciniert wird.

Insbesondere bei Verwendung des Katalysators in einem Festbett bei der Epoxidierung von Olefinen bzw. der Ammoximierung von Ketonen ist es erstrebenswert, größere Formkörper auszubilden, die eine ausreichende Härte und Abriebfestigkeit aufweisen, damit die physikalische Integrität der Katalysatorformkörper auch nach längerem Betrieb bestehen bleibt. Insbesondere soll die Bildung von Feinkorn, das mit dem Reaktionsprodukt ausgetragen wird, was zum einen zu Katalysatorverlusten führt und zum anderen eine aufwendigere Auftrennung der Reaktionsprodukte notwendig macht, verringert bzw. vermieden werden.

Aus DE-A 196 23 611 ist ein Verfahren zur Herstellung von Titansilicalitformkörpern bekannt, nach dem zuerst ein Synthesegel durch Hydrolyse einer Siliciumquelle, einer Titanquelle und Tetrapropylammoniumhydroxid hergestellt wird, dieses Synthesegel unter hydrothermalen Bedingungen kristallisiet wird, der entstandene Feststoff z. B. durch Zentrifugation abgetrennt wird, getrocknet und anschließend bei 550°C calciniert wird. Dieses calcinierte Titansilicalitpulver wird dann anschließend durch Zugabe von Wasser, Bindemittel wie z. B. Kieselsol und Plastifizierungsmittel wie Methylcellulose zu einer plastischen Masse weiterverarbeitet, die zu Strängen extrudiert wird, diese Stränge getrocknet und noch einmal bei 500°C 5 h lang calciniert werden. Die Stränge werden dann zu Granulat oder Split mit einer Korngröße zwischen 1 und 10 mm zerkleinert.

WO 98/55229 offenbart ein vergleichbares Verfahren bei dem die verformbare Masse durch Zugabe einer Mischung von Wasser und Alkohol als Anteigungsmittel hergestellt wird.

Trotz dieser aus dem Stand der Technik bekannten Verfahren zur Herstellung von Titansilicalitformkörpern besteht nach wie vor ein Bedürfnis in der Industrie, solche Verfahren zu modifizieren, um Formkörper mit verbesserten mechanischen Eigenschaften zu erhalten. Aufgabe der vorliegenden Erfindung ist es daher, ein Verfahren zur Herstellung von Titansilicalitformkörpern bereitzustellen, das zu Formkörpern mit verbesserten mechanischen Eigenschaften führt. Eine weitere Aufgabe der vorliegenden Erfindung ist es, einen Titansilicalitformkörper mit verbesserten Eigenschaften bereitzustellen, der auch als Katalysator in Epoxydierungsreaktionen von Olefinen sowie in Ammoximierungsreaktionen von Ketonen einsetzbar ist.

Diese Aufgabe wird durch ein Verfahren zur Herstellung eines Titansilicalitkörpers durch
a) Ausbildung einer formbaren Masse, enthaltend Titansilicalit, ein oder mehrere Bindemittel, so daß die Curdkurve der verformbaren Masse einen Plateauwert im Bereich von 20 bis 90 mm aufweist,
b) Verformen der Masse aus Schritt a) zu einem Grünkörper,
c) eventuell Trocknen und
d) Calcinieren des Grünkörpers,
sowie durch einen nach diesem Verfahren erhältlichen Titansilicalitformkörper gelöst.

Es wurde überraschenderweise festgestellt, daß, wenn die verformbare Masse so ausgebildet wird, daß deren Curdkurve einen Plateauwert im Bereich von 20 bis 90 mm aufweist, die resultierenden Titansilicalitkörper verbesserte mechanische Eigenschaften wie z. B. eine höhere Bruchfestigkeit aufweisen.

Der Plateauwert in der Curdkurve der verformbaren Masse hängt unter vorgegebenen Meßbedingungen von einer Vielzahl unterschiedlicher Faktoren wie z. B. Korngröße der verwendeten Titansilicalitkristalle, Art und Menge des Bindemittels, Art und Menge des Anteigungsmittels, falls vorhanden, Art und Menge von Formgebungshilfsmitteln usw.ab. Somit ist es nicht möglich, für alle diese Faktoren genau Bereiche anzugeben, innerhalb derer ein Plateauwert der Curdkurve im erfindungsgemäßen Bereich erreicht wird.

Der Fachmann in Kenntnis der oben angegebenen Faktoren, die den Plateauwert der Curdkurve beeinflussen, ist aber ohne weiteres in der Lage, durch einfache Standardversuche zu bestimmen, wie die einzelnen Faktoren ausgewählt werden müssen, um den erfindungsgemäßen Curdwert zu ergeben.

Die Meßmethode zur Erstellung der Curdkurve und die Bestimmung des Plateauwerts aus der Curdkurve ist detailliert in der Bedienungsanleitung des Curd Meter Modells M-301AR der "lio Electric Co., Ltd., 2-23-2, (1140) YOYOGI, SHIBUYA-KU, TOYKYO, Japan" angegeben.

Besonders geeignete Titansilicalitformkörper mit hoher Seitenbruchfestigkeit werden erhalten, wenn die verformbare Masse einen Plateauwert der Curdkurve im Bereich von 30 bis 70 mm aufweist.

Die erfindungsgemäßen Titansilicalitkörper sind insbesondere geeignet als Katalysatoren in Festbettreaktoren für die Oxydierung organischer Verbindungen, insbesondere für die Epoxidierung von Olefinen und die Ammoximierung von Ketonen.

Der Titansilicalit, der zur Ausbildung der verformbaren Masse gemäß des erfindungsgemäßen Verfahrens verwendet wird, kann durch übliche aus dem Stand der Technik bekannte Verfahren hergestellt werden. Hierzu wird eine SiO₂-Quelle, eine TiO₂-Quelle, eine Templatverbindung sowie Wasser zusammengegeben. Insbesondere eignen sich hydrolysierbare Siliciumverbindungen und hydrolysierbare Titanverbindungen als Ausgangsverbindung, die dann in Gegenwart von Wasser hydrolysiert werden. Geeignete hydrolysierbare Silicium- bzw. Titanverbindungen sind Tetraalkylorthosilicate bzw. Tetraalkylorthotitanate, wobei Alkyl vorzugsweise ausgewählt ist aus der Gruppe bestehend aus Methyl, Ethyl, Propyl oder Butyl. Die am meisten bevorzugten Ausgangsverbindungen sind Tetraethylorthosilicat und Tetraethylorthotitanat. Unter Templatverbindungen versteht man Verbindungen, die durch Aufnahme in das Kristallgitter des Produkts während der Kristallisation die Kristallstruktur bestimmen. Bevorzugte Templatverbindungen sind quartäre Ammoniumverbindungen wie Tetraalkylammoniumverbindungen, insbesondere Tetraalkylammoniumhydroxid wie Tetra-n-propylammoniumhydroxid zur Herstellung von Titansilicalit-1 (MFI-Struktur), Tetra-n-butylammoniumhydroxid zur Herstellung von Titansilicalit-2 (MEL-Struktur) und Tetraethylammoniumhydroxid zur Herstellung von Titan-β-zeolith (DEA-Kristallstruktur).

Der für die Synthese erforderliche pH-Wert des Synthesegels von > 9, bevorzugt > 11 stellt sich durch die basisch reagierende quartäre Ammoniumverbindung, die als Templat eingesetzt wird, ein. Die Temperatur, bei der das Synthesegel hergestellt wird, kann in weiten Grenzen variiert werden, bevorzugt ist allerdings das Gemisch aus Siliciumquelle, Titanquelle auf eine Temperatur im Bereich von 0°C bis 10°C bis vorzugsweise 0°C bis 5°C, besonders bevorzugt 1°C abzukühlen und dann die Templatverbindung in wäßriger Lösung, die auf die gleiche Temperatur abgekühlt ist, zuzugeben.

In einer weiteren Ausführungsform der vorliegenden Erfindung wird bei der Verwendung von Tetraalkylorthosilicaten und Tetraalkylorthotitanaten als Silicium- bzw. Titanquelle das Synthesegel auf eine Temperatur von 75°C bis 95°C für eine Zeitdauer von 120 bis 200 min erwärmt und der entstehende Alkohol als Wasserazeotrop abdestilliert, um die Hydrolyse der hydrolysierbaren Titan- und Siliciumverbindungen zu unterstützen. Das Synthesegel wird anschließend gegebenenfalls nach einer zusätzlichen Reifezeit bei einer Temperatur von 150°C bis 220°C, vorzugsweise 170°C bis 190°C unter autogenem Druck kristallisiert. Unter den vorgegebenen Bedingungen beträgt die Kristallisationszeit in der Regel weniger als 3 Tage, vorzugsweise weniger als 24 h.

Ebenfalls gut geeignete Ausgangsverbindungen sind Silicium-Titan-Mischoxide, die beispielsweise durch Flammenhydrolyse eines Gemisches von SiCl₄ und TiCl₄ hergestellt werden können. Diese können in einer geeigneten Templat- und Base-enthaltenden Lösung dispergiert und nach einem evtl. Alterungsschritt bzw. der Zugabe von Impfkristallen wie oben beschrieben kristallisiert werden.

Die so erhaltene Titansilicalitsuspension kann in unterschiedlicher Art und Weise weiterverarbeitet werden. Zum einen ist es möglich, die Titansilicalitkristalle durch die üblichen Fest/Flüssig-Trennverfahren wie Zentrifugation und Filtration von der Mutterlauge abzutrennen, zu trocknen und zu calcinieren. Alternativ kann der Titansilicalitfeststoff durch Sprühtrocknen oder mittels Wirbelschichtsprühgranulationstrocknung mit oder ohne anschließender Calcinierung erhalten werden. Entsprechend einer weiteren Ausführungsform kann die Kristallitsuspension evtl. aufkondensiert werden, z. B. durch Verdampfen leichtflüchtiger Bestandteile oder durch Hinzufügen von Titansilicalitfeststoff. Die Titansilicalitsuspension kann dann direkt mit einem Bindemittel und falls erforderlich Anteigungsmittel und weiteren Hilfsmitteln vermischt werden, um die verformbare Masse gemäß des erfindungsgemäßen Verfahrens herzustellen.

Nach der Trocknung wird der Titansilicalitfestkörper bzw. die aufkonzentrierte Titansilicalitsuspension mit einem oder mehreren Bindemitteln, gegebenenfalls mit Formgebungshilfsmittel und gegebenenfalls einem Anteigungsmittel zu einer verformbaren Masse verarbeitet. Als Bindemittel eignen sich im Prinzip alle für derartige Zwecke bekannten Stoffe. Bevorzugt werden Oxide des Siliciums, Aluminiums, Bors, Phosphors, Titans, Zirkoniums und/oder Magnesiums bzw. entsprechende Vorstufen verwendet. Besonders bevorzugte Bindemittel sind Siliciumdioxide, Aluminiumoxide, Titanoxide und Tonmineralien sowie Mischungen hiervon. Beispiele für Siliciumdioxide bzw. Siliciumdioxid-Vorstufen sind gefällte oder pyrogene Kieselsäuren, Kieselsole, Tetraalkoxysilane, teil kondensierte Kieselsäureester und Polysiloxane, für Aluminiumoxid (Pseudo-) Boehmit, für Titandioxid Anatas oder Brookit und für Tonmineralien Montmorillonite, Kaoline, Bentonite und Sepiolithe.

Die Bindemittel können als Pulver oder in Form von Solen, Suspensionen oder Lösungen verwendet werden. Die Menge an Bindemittel beträgt im allgemeinen 1 - 99 Gew.-%, vorzugsweise 5 - 60 Gew.-% und besonders 10 - 40 Gew.-% bezogen auf den Feststoffgehalt der verformbaren Masse.

Ganz besonders geeignete Bindemittel sind Aluminiumoxide, Polysiloxane, Kieselsole, Tonmineralien sowie die Kombination von SiO₂ und Borverbindungen. Hierbei hat sich herausgestellt, daß insbesondere mit Aluminiumoxid, Tonmineral bzw. der Kombination von Kieselsol und Borverbindung auch unabhängig vom Einhalten bestimmter Plateauwerte der Curdkurve bei der Herstellung der verformbaren Masse Titansilicalitformkörper mit hervorragenden mechanischen Eigenschaften, insbesondere hoher Bruchfestigkeit erhalten werden. Besonders bevorzugt ist eine Mischung von Kieselsol und Borverbindung als Bindemittel zur Herstellung von Titansilicalitformkörpern. Als Borverbindung ist insbesondere Borsäure gut geeignet. In diese bevorzugte Mischung aus Kieselsol und Borverbindung liegt das Kieselsol in einer Menge von 50 - 99,99 Gew.-%, bezogen auf die Gesamtmasse von Kieselsol und Borverbindung, vor.

Als Formgebungshilfsmittel können Substanzen zugesetzt werden, die in erster Linie die Bildung einer plastischen Masse während des Knetverformens und Trockenschritts und darüber hinaus die mechanische Stabilität des Formkörpers beim Verformen und Trocknen begünstigen. Diese Substanzen werden vorzugsweise beim Calcinieren des Grünkörpers entfernt. Bevorzugt sind organische viskositätssteigernde Substanzen, wie z. B. Cellulose, Stärke, Acrylat, Polyacrylat, Polymethacrylat, Polyvinylalkohol, Polyvinylpyrolidon, Polyisobuten und Polytetrahydrofuran, Polyglykolether, Fettsäureverbindungen und/oder Wachsemulsionen.

Als weitere Zusatzstoffe können Basen oder Säuren zugesetzt werden. Geeignet sind beispielsweise Ammoniak, Amine oder quarternäre Ammoniumsalze sowie Carbonsäuren, wie z. B. Ameisen-, Essig- oder Propionsäure. Selbstverständlich können auch Gemische aus zwei oder mehr der oben genannten Zusatzstoffe verwendet werden.

Als weitere Zusatzstoffe können Porenbildner, wie Polyalkohole, Fructose, Pentaerythrit, Cellulose oder Sägemehl eingesetzt werden. Diese brennen bei der Calcinierung heraus und hinterlassen zusätzliche Meso- und Makroporen im Formkörper.

Bei Verwendung eines Zweischneckenextruders können die Komponenten direkt vor dem Extrudieren gemischt werden. Formgebungshilfsmittel werden in dem erfindungsgemäßen Verfahren in einer Menge von 0 bis 40 Gew.-%, vorzugsweise 1 - 10 Gew.-%, bezogen auf den Festkörpergehalt der verformbaren Masse, eingesetzt.

Als Anteigungsmittel wird vorzugsweise in dem erfindungsgemäßen Verfahren ein wäßriges Medium verwendet, das gegebenenfalls ein wassermischbares organisches Lösungsmittel enthält. Wenn als Bindemittel ein Metallsäureester wie z. B. Alkylorthosilicat oder Alkylorthotitanat verwendet wird ist es bevorzugt, als Anteigungsmittel eine Mischung aus Wasser und einem Alkohol, der der Alkoholkomponente des Metallsäureesters entspricht, zu verwenden. Bei der Verwendung eines Gemisches aus Alkohol und Wasser als Anteigungsmittel beträgt der Alkoholgehalt dieser Mischung im allgemeinen 1 bis 80 Gew.-%, vorzugsweise 5 bis 70 Gew.-% und insbesondere bevorzugt 10 bis 60 Gew.-%, jeweils bezogen auf das Gesamtgewicht der Mischung.

Der verformbaren Masse können außerdem beliebige weitere Komponenten, wie z. B. weitere Zeolithe, pyrogene bzw. gefällte Oxide und Metalle, sowie zur Verbesserung der mechanischen Stabilität, anorganische Fasern, wie z. B. Al-Si-Fasern und/oder Ca-Si-Fasern zugegeben werden.

Das Anteigungsmittel wird in Mengen eingesetzt, so daß eine plastische extrudierbaree Masse entsteht, vorzugsweise in einer Menge, so daß der Festkörpergehalt der verformbaren Masse 10 - 90 Gew.-%, vorzugsweise 60 - 80 Gew.-% beträgt.

Die Zugabenreihenfolge der Bestandteile der verformbaren Masse hängt von einer Reihe von Faktoren ab und muß im Einzelfall ausgearbeitet werden. Es ist sowohl möglich, zuerst zu dem Titansilicalitfeststoff das Bindemittel zuzugeben, anschließend die gegebenenfalls verwendeten Formgebungshilfsmittel und zum Schluß das Anteigungsmittel zuzugeben. Nach der Zugabe des Bindemittels und gegebenenfalls des Formgebungshilfsmittels wird die in der Regel noch pulverförmige Masse im Kneter, Extruder oder Mischer homogenisiert und dann das Anteigungsmittel zugesetzt. Die so erhaltene Mischung wird solange gemischt, bis eine verstrangbare oder extrudierbare plastische Masse entstanden ist. Dabei wird in der Regel bei Temperaturen im Bereich von 10°C bis zum Siedepunkt des Anteigungsmittels und unter Normaldruck, leichtem überatmosphärischem Druck oder Unterdruck gearbeitet. Die Auswahl und die Mengen der verwendeten Substanzen sind so abzustimmen, daß die verformbare Masse einen Plateauwert der Curdkurve im erfindungsgemäßen Bereich aufweist.

Bei der Ausführungsform, die von der aufkonzentrierten Titansilicalitsuspension ausgeht, wird vorzugsweise das Bindemittel und gegebenenfalls Formgebungshilfsmittel der Titansilicalitsuspension zugegeben und falls erforderlich, um eine knetbare Masse zu ergeben, zusätzlich ein Anteigungsmittel zugegeben.

Es können für die Mischung und Verformung alle bekannten Misch- und Verformungsvorrichtungen bzw. -verfahren verwendet werden. Derartige bekannte Verformungsvorrichtungen werden beispielsweise in Ullmann's Enzyklopädie der Technischen Chemie, 4. Auflage, Band 2, Seite 295 ff., 1972 beschrieben. Bevorzugt werden Ein- und Zweischneckenextruder oder eine Strangpresse eingesetzt. Dabei können zahlreiche bekannte Geometrien, wie z. B. Vollzylinder, Hohlzylinder, Sterne usw. hergestellt werden. Möglich ist jedoch auch die Herstellung von Wabenkörpern.

Die so erhaltenen Grünkörper, deren Durchmesser zwischen 1 und 10 mm liegt können anschließend bei Raumtemperatur oder erhöhten Temperaturen, ggf. in Gegenwart von Wasserdampf, getrocknet werden.

Anschließend werden die Formkörper in Gegenwart von Luft, Inertgas oder Sauerstoff bei Temperaturen bis zu 1.100°C calciniert. Der zeitliche Verlauf der Temperatur, d. h. Aufheizrate, Haltezeiten bei Zwischen- und Endtemperatur, sowie Abkühlrate müssen dabei im Einzelfall optimiert werden. Die Kalzinierung kann zur Entfernung des Templats und der Zusatzstoffe aus dem Grünkörper dienen und beeinflußt in entscheidender Weise die mechanische Stabilität, das Porenvolumen oder die spezifische Oberfläche.

Die erhaltenen Stränge bzw. Extrudate können zerkleinert werden. Sie werden dabei vorzugsweise zu einem Granulat oder Splitt mit einem Partikeldurchmesser von 0,1 bis 5 mm, insbesondere von 0,5 bis 2 mm zerkleinert.

Zur Verdeutlichung der vorliegenden Erfindung werden die folgenden Beispiele angegeben:

### Verwendete Apparaturen für die Herstellung der Formmassen

Die Formmassen wurden mit auf 10°C thermostatisierten 0,25-1- bzw. 2,5-1-Knetern der Firma Werner & Pfleiderer (Typ UK 0,25 bzw. LUK 2,5) hergestellt. Beide Kneter waren mit Sigmaschaufeln ausgerüstet, wobei der 2,5-1-Kneter zusätzlich über eine regelbare Austragsschnecke verfügte. Im 0,25-1-Kneter hergestellte Formmassen wurden mit einem Handextruder mit 4-mm-Lochplatte verformt. Im 2,5-1-Kneter mit Austragsschnecke erzeugte Knetmassen wurden mit Hilfe der Austragungsschnecke extrudiert (4 mm Vollzylinder).

### Charakterisierung der Formmassen

Um die Formmassen vor der Formgebung zu charakterisieren, wurde die Höhe des Plateauwerts der Curdkurve (Plateauwert) bestimmt. Dazu wurde ein Probengefäß mit einem Durchmesser von 50 mm und einer Höhe von 40 mm vollständig mit der zu untersuchenden Formmasse gefüllt. Mit einer Presse wurde die Formmasse 5 min mit einem Druck von 10 bar belastet, um den Einfluß der Scherung während des Knetens zu eliminieren.

Anschließend wurde das Probengefäß auf die Grundplatte eines Curdmeters M-301AR der Firma Iio Electric Col, Ltd. gestellt. Die Grundplatte kann mittels eines Elektromotors mit exakt einstellbarer Geschwindigkeit angehoben werden. Der an einer Präzisionsfeder (Federkonstante 6533 dyn/cm) montierte Meßstab mit einem Durchmesser von 2 mm befand sich dabei direkt über der Mitte des Probengefäßes. Über dem Meßstab wurden außerdem 400-g-Gewichte aufgelegt.

Dann wurde die Grundplatte mit dem Probengefäß so weit angehoben, bis die Spitze des Meßstabes direkt auf der Knetmasse auflag. Die ebenfalls über den Elektromotor angetriebene Schreibtrommel mit dem aufgespannten Schreibpapier wurde eingelegt und der Schreibstift mit Hilfe der Nullpunkteinstellung justiert.

Für die eigentliche Messung wurde die Grundplatte mit der darauf befindlichen Formmasse mit einer konstanten Geschwindigkeit von 0,36 cm/s angehoben. Auf der synchron dazu drehenden Schreibtrommel wurde der zeitliche Verlauf der Höhe des Meßstabes aufgezeichnet. Zu Beginn der Messung wurde der Meßstab durch die Knetmasse mit angehoben, bis er nach Erreichen einer bestimmten Höhe in die Knetmasse hineingedrückt wurde. Die Curdkurve verläuft somit zunächst parallel zur Flächendiagonale, bis sie abknickt und parallel zur x-Achse verläuft. Der Abstand zur x-Achse, die Plateauhöhe der Curdkurve in mm (Plateauwert), wurde zur Charakterisierung der Knetmasse herangezogen.

### Bestimmung der Seitenbruchfestigkeit der calcinierten Formkörper

Die Seitenbruchfestigkeit der calcinierten Formkörpers wurde mittels eines Tablettenbruchfestigkeits-Testers (TBH 28 Erweka) durch Mittelwertbildung von insgesamt 20 Messwerten für jede Probe bestimmt.

### Bezugsbeispiel 1

### Herstellung von Titansilicalit

In einem mit Stickstoff inertisierten 10-1-Autoklaven wurden Tetraethylorthosilicat, Tetraethylorthotitanat und Tetra-n-propylammoniumhydroxid in wäßriger Lösung zusammengegeben und hydrolysiert. Die Mengen wurden dabei so gewählt, daß das molare Verhältnis Si/Ti = 35 war, das molare Verhältnis von N/Si = 0,17 und das molare Verhältnis von H₂O/Si = 27 betrug. Nach Beendigung der Hydrolyse und Abdestillieren des gebildeten Alkohols, der durch das gleiche Volumen Wasser ersetzt wurde, wurde das Synthesegel 3 h lang bei 175°C unter Hydrothermalbedingungen kristallisiert. Der gebildete Feststoff wurde abgetrennt, gewaschen und bei 110°C über Nacht getrocknet. Ein Teil des getrockneten Produkts wurde außerdem bei 550°C 4 h lang in Luftatmosphäre calciniert.

### Beispiel 1

In dem 2,5-1-Kneter mit Austragschnecke wurden 1.027,6 g calciniertes TS-1-Pulver aus Bezugsbeispiel 1, 26,0 g Methylhydroxycellulose (Tylose MH 1000, Firma Hoechst) und 333,3 g Aluminiumoxid (Plural SB, Firma Condea) eingewogen, 10 min gemischt und anschließend mit 30,0 g Eisessig (Firma Merck) versetzt. Danach wurden innerhalb von 20 min insgesamt 551,3 g VE-Wasser zugegeben, wobei sich eine kompakte Masse bildete, die weitere 45 min geknetet wurde.

Diese Formmasse wurde wie oben beschrieben charakterisiert. Es ergab sich ein Plateauwert der Curdkurve von 54mm.

Die Formmasse wurde mittels einer Austragungsschnecke mit einem Mundstück mit 4-mm-Bohrung extrudiert. Die entstandenen Grünkörper wurden mit einem Leister-Gebläse angetrocknet, über Nacht an der Luft stehengelassen und bei 550°C 1 h lang in einem Kammerofen calciniert (Aufheizrate 100 k/h). Die mittlere Seitenbruchfestigkeit der Formkörper betrug 87 N.

### Beispiel 2

In einem 20-1-Kneter wurden 1800 g getrocknetes TS-1-Pulver aus Bezugsbeispiel 1, 100 g Tylose MH1000, 1.360 g Plural SB, 150 g Eisessig und 1.880 g VE-Wasser vorgelegt und 15 min gemischt. Innerhalb von 10 min wurden unter Kneten portionsweise weitere 3.000 g TS-1-Pulver und nach 10 min 40 g Zusoplast 126/3 (Fettsäurezubereitung mit nichtionischem Emulgator, Firma Zschimmer & Schwarz) zugegeben. Die resultierende Masse wurde weitere 60 min geknetet.

Der Plateauwert der Curdkurve der Formmasse betrug 81 mm.Die Formmasse wurde wie in Beispiel 1 extrudiert. Die Seitenbruchfestigkeit der resultierenden Formkörper betrug 46 N.

### Beispiel 3

In einem 2,5-1-Kneter mit Austragschnecke wurden 646,4 g calciniertes TS-1-Pulver aus Beispiel 1, 32,0 g Tylose MH1000 und 218,4 g Pural SB vorgelegt und 10 min gemischt. Zu dieser Mischung wurde eine Lösung von 24,0 g Eisessig in 463,5 g VE-Wasser gegeben und weitere 60 min geknetet. Anschließend wurden der Masse unter weiterem Kneten 6,4 g Zusoplast 126/3 und nach weiteren 30 min 20 g Petroleum zugesetzt.

Der Plateauwert der Curdkurve der Formmasse betrug 59 mm. Die Formmasse wurde wie in Beispiel 1 extrudiert. Die Seitenbruchfestigkeit der resultierenden Formkörper betrug 103 N.

### Vergleichsbeispiel 1

102,8 g calciniertes TS-1-Pulver aus Beispiel 1, 25 g Pural SB, 2,8 g Eisessig und 2,5 g Tylose MH1000 wurden in einem 0,25-1-Kneter vorgelegt und 10 min gemischt. Dann wurden unter Kneten 7,5 g Orthophosphorsäure (85 Gew.-%, Firma Merck) und innerhalb von 50 min 67,1 g Wasser portionsweise zugegeben. Um die Thixotropie der Masse zu reduzieren, wurden anschließend langsam 11,8 g Ammoniaklösung (25 Gew. -%) zugegeben.

Der Plateauwert der Curdkurve der Formmasse betrug 94 mm. Die Formmasse wurde wie in Beispiel 1 beschrieben weiterverarbeitet. Die Seitenbruchfestigkeit der Formkörper betrug 7 N.

### Vergleichsbeispiel 2

120,1 g getrocknetes TS-1-Pulver aus Beispiel 1, 34,7 g Pural SB, 3,9 g Eisessig und 2,6 g Tylose MH1000 wurden im 0,25-1-Kneter vorgelegt und 10 min gemischt. Nach Zugabe von 63,0 g Wasser wurde die resultierende Masse weitere 30 min geknetet.

Der Plateauwert der Curdkurve der Formmasse betrug 19 mm. Die Formmasse wurde wie in Beispiel 1 beschrieben weiterverarbeitet. Die Seitenbruchfestigkeit der Formkörper betrug 19 N.

Aus dem Vergleich zwischen den Beispielen 1-3 mit den Vergleichsbeispielen 1 und 2, die alle Aluminiumoxid als Bindemittel verwenden, wird deutlich, daß die Seitenbruchfestigkeit der resultierenden calcinierten Formkörper stark von den plastischen Eigenschaften der Formmasse abhängt und Formkörper mit einer hohen Seitenbruchfestigkeit aus Formmassen mit einem Plateauwert der Curdcurve im erfindungsgemäßen Bereich erhalten werden.

### Beispiel 4

In einem 10-1-Reaktor wurden 3.610,6 g Tetraethylorthosilicat vorgelegt und auf 1°C abgekühlt. Unter Rühren wurde innerhalb von 4 h eine Mischung von 3.151,1 g einer wässrigen Tetra-n-propylammoniumhydroxidlösung (40 Gew.-%, Firma Sachen) und 1.229,3 g entionisiertem Wasser zudosiert. Die Reaktionsmischung wurde anschließend auf max. 95°C erhitzt, um 5.140 ml eines Wasser-Ethanol-Azeotrops abzudestillieren. (TPA-Silicat-Lösung)

102,75 g calciniertes TS-1-Pulver aus Bezugsbeispiel 1 und 2 g Tylose MH1000 wurden im 0,25-1-Kneter vorgelegt und mit 128,2 g der oben hergestellten TPA-Silicat-Lösung versetzt. Nach 10-minütigem Mischen wurden tropfenweise 4,2 g Eisessig und anschließend 6,3 g Wasser zugegeben.

Der Plateauwert der Curdkurve der Formmasse betrug 33 mm. Die Formmasse wurde mittels eines Handextruders mit 4-mm-Lochplatte extrudiert. Die Grünkörper wurden wie in Beispiel 1 beschrieben weiterverarbeitet. Die Seitenbruchfestigkeit der erhaltenen Formkörper betrug 32 N.

### Vergleichsbeispiel 3

Zur Herstellung einer Formmasse wurden 119,05 g getrocknetes TS-1-Pulver aus Beispiel 1 und 2 g Tylose MH1000 in einem 0,25-1-Kneter vorgelegt, und 128,2 g der wie in Beispiel 4 hergestellten TPA-Silicatlösung zugesetzt. Nach 10-minütigem Mischen wurden 9,0 g Ammoniumacetat zugegeben, und die resultierende Formmasse wurde weitere 20 min geknetet.

Der Plateauwert der Curdkurve der Formmasse betrug 18 mm. Die Formmasse wurde mittels eines Handextruders mit 4-mm-Lochplatte extrudiert. Die Grünkörper wurden wie in Beispiel 1 beschrieben weiterverarbeitet. Die Seitenbruchfestigkeit des calcinierten Formkörpers war so gering, daß sie nicht gemessen werden konnte.

### Vergleichsbeispiel 4

In einem 2,5-1-Kneter mit Austragsschnecke wurden 822 g calciniertes TS-1-Pulver aus Beispiel 1, 16,0 g Tylose MH1000 und 1.025,8 g der wie in Beispiel 4 hergestellten TPA-Silicat-Lösung gemischt. Unter Kneten wurden dann innerhalb von 2 h insgesamt 28,4 g Eisessig langsam zugegeben.

Der Plateauwert der Curdkurve der Formmasse betrug 14 mm. Die Formmasse wurde mit dem Kneter mit Austragsschnecke extrudiert. Die Grünkörper wurden wie oben beschrieben weiterverarbeitet. Die Seitenbruchfestigkeit der erhaltenen Formkörper betrug 10 N.

### Beispiel 5

102,75 g calciniertes TS-1-Pulver aus Bezugsbeispiel 1 und 2 g Tylose MH1000 wurden im 0,25-1-Kneter vorgelegt und mit 62,5 g Kieselsol Ludox AS40 gemischt. Anschließend wurden 49,7 g Wasser und 1,8 g Ammoniaklösung (25 Gew.-%) zugegeben. Die resultierende Masse wurde insgesamt 2 h geknetet.

Der Plateauwert der Curdkurve der Formmasse betrug 37 mm. Die Formmasse wurde mittels eines Handextruders mit 4-mm-Lochplatte extrudiert. Die Grünkörper wurden wie in Beispiel 1 beschrieben weiterverarbeitet. Die Seitenbruchfestigkeit der erhaltenen Formkörper betrug 21 N.

### Vergleichsbeispiel 5

119,05 g getrocknetes TS-1-Pulver aus Beispiel 1 und 2 g Tylose MH1000 wurden im 0,25-1-Kneter vorgelegt und mit 62,5 g Kieselsol Ludox AS40 gemischt. Nach Zugabe von 18,6 g Wasser wurde die resultierende Masse insgesamt 40 min geknetet.

Der Plateauwert der Curdkurve der Formmasse betrug 14 mm. Die Formmasse wurde mittels eines Handextruders mit 4-mm-Lochplatte extrudiert. Die Grünkörper wurden wie in Beispiel 1 beschrieben weiterverarbeitet. Die Seitenbruchfestigkeit der erhaltenen Formkörper betrug 15 N.

### Beispiel 6

In einem 2,5-1-Kneter mit Austragschnecke wurden 1.063,3 g calciniertes TS-1-Pulver aus Beispiel 1, 26,0 g Tylose MH1000 und 618,8 g Kieselsol Ludox AS 40 vorgelegt und 10 min gemischt. Anschließend wurden 200 g Wasser und 16,5 g Ammoniaklösung (25 Gew.-%) zugegeben. Die resultierende Masse wurde anschließend weitere 3 h geknetet.

Der Plateauwert der Curdkurve der Formmasse betrug 67 mm. Die Formmasse wurde mittels der Austragschnecke extrudiert. Die Grünkörper wurden wie in Versuch TS 22 beschrieben weiterverarbeitet. Die Seitenbruchfestigkeit der erhaltenen Formkörper betrug 21 N.

### Beispiel 7

102,8 g calciniertes TS-1-Pulver aus Beispiel 1, 11,1 g Borsäure (99,5 %, Firma Merck), 2,0 g Tylose MH15000 (Firma Hoechst) und 46,9 g Kieselsol Ludox AS40 (Firma DuPont) wurden in einem 0,25-1-Kneter eingewogen und gemischt. Innerhalb von 1 h wurden während des Mischens 3,0 g wässrige Ammoniaklösung (25 Gew.-%) und 42,5 g Wasser portionsweise zugesetzt. Anschließend wurde die resultierende Masse weitere 10 min geknetet.

Der Plateauwert der Curdkurve der Formmasse betrug 45 mm. Die Formmasse wurde mittels eines Handextruders mit 4-mm-Lochplatte extrudiert. Die Grünkörper wurden wie in Beispiel 1 beschrieben weiterverarbeitet. Die mittlere Seitenbruchfestigkeit der resultierenden Formkörper betrug 57 N.

Der Vergleich zwischen Beispiel 5 und Beispiel 7 zeigt, daß vergleichbare Gesamtmengen von Bindemitteln und Mengen der anderen Bestandteile zu ähnlichen Plateauwerten der Curdkurve führen, der Zusatz von Borsäure als Bindemittelkomponente aber zusätzlich eine deutlichen Steigerung der Seitenbruchfestigkeit des resultierenden Formkörpers bewirkt. Weiterhin wird aus dem Vergleich der Beispiele gemäß der vorliegenden Erfindung deutlich, daß die Verwendung von Aluminiumoxid und der Kombination von Kieselsol und Borverbindung als Bindemittel zu den höchsten Werten für die Seitenbruchfestigkeit der resultierenden Formkörper führt.

## Patentansprüche

1. Verfahren zur Herstellung eines Titansilicalitformkörpers durch
a) Ausbilden einer verformbaren Masse enthaltend Titansilicalit, ein Bindemittel und ein Anteigungsmittel, so daß die Curdkurve der verformbaren Masse einen Plateauwert im Bereich von 20 bis 90 mm aufweist,
b) Verformen der Masse aus Schritt a) zu einem Grünkörper,
c) eventuell Trocknen und
d) Calcinieren des Grünkörpers.

2. Verfahren nach Anspruch 1, wobei
die Curdkurve der verformbaren Masse einen Plateauwert im Bereich von 25 bis 70 mm aufweist.

3. Verfahren nach einem der vorstehenden Ansprüche, wobei
als Bindemittel Verbindungen des Siliciums, Aluminiums, Bors, Phosphors, Zirkoniums und/oder Titans verwendet werden.

4. Verfahren nach Anspruch 3, wobei
das Bindemittel ausgewählt ist aus Aluminiumoxid, Siliciumoxid, hydrolisierbaren Siliciumverbindungen und partiellen oder vollständigen Hydrolysaten hiervon, Borverbindungen, Phosphorverbindungen, Tonmineralien und Mischungen hiervon.

5. Verfahren nach Anspruch 4, wobei
das Bindemittel Aluminiumoxid enthält.

6. Verfahren zur Herstellung eines Titansilicalitformkörpers durch
a) Ausbilden einer verformbaren Masse enthaltend Titansilicalit, Aluminiumoxid und ein Anteigungsmittel,
b) Verformen der Masse aus Schritt a) zu einem Grünkörper,
c) eventuell Trocknen und
d) Calcinieren des Grünkörpers.

7. Verfahren nach Anspruch 4, wobei
das Bindemittel ein Tonmineral enthält.

8. Verfahren nach Anspruch 4, wobei
das Bindemittel eine Kombination von Kieselsol und einer Borverbindung enthält.

9. Verfahren zur Herstellung eines Titansilicalitformkörpers durch
a) Ausbilden einer verformbaren Masse enthaltend Titansilicalit, eine Kombination von Kieselsol und einer Borverbindung als Bindemittel und ein Anteigungsmittel,
b) Verformen der Masse aus Schritt a) zu einem Grünkörper,
c) eventuell Trocknen und
d) Calcinieren des Grünkörpers.

10. Verfahren nach einem der Ansprüche 8 oder 9, wobei
die Borverbindung Borsäure ist.

11. Verfahren nach einem der Ansprüche 8-10, wobei
Kieselsol in einer Menge von 50 bis 99,99 Gew.-% in der Mischung aus Kieselsol und Borverbindung vorhanden ist.

12. Verfahren nach einem der vorstehenden Ansprüche, wobei
als Anteigungsmittel ein wäßriges Medium verwendet wird, das gegebenenfalls ein wassermischbares organisches Lösungsmittel enthält.

13. Verfahren nach einem der vorstehenden Ansprüche, wobei
der Grünkörper bei einer Temperatur von 400°C bis 1000°C, vorzugsweise von 500°C bis 750°C calciniert wird.

14. Titansilicalitformkörper erhältlich nach einem Verfahren gemäß einem der vorstehenden Ansprüche.

15. Verfahren zur Epoxidierung von Olefinen mit wässrigem Wasserstoffperoxid in Gegenwart von Titansilicalitformkörpern nach Anspruch 14.

16. Verfahren zur Ammoximierung von Ketonen mit wässrigem Wasserstoffperoxid und Ammoniak in Gegenwart von Titansilicalitformkörpern nach Anspruch 14.
